# EUROPEAN PATENT SPECIFICATION

(11) **EP 0 677 301 B1**
(45) Date of publication and mention of the grant of the patent: **05.07.2000**
(21) Application number: 95850075.3
(22) Date of filing: 11.04.1995
(51) Int. Cl.: A61N 1/05

(54) **Electrode apparatus with a variable distance between the electrodes**
Elektrodenanordnung mit einem zwischen den Elektroden variablen Abstand
Ensemble d'électrodes avec un éloignement intermédiaire variable des électrodes

(30) Priority: 14.04.1994 SE 9401267
(43) Date of publication of application: 18.10.1995
(73) Proprietor: Pacesetter AB, 175 84 Järfälla (SE)
(72) Inventor: Ekwall, Christer, S-163 57 Spanga (SE); Högnelid, Kurt, S-137 38 Västerhaninge (SE)
(74) Representative: Lettström, Richard Wilhelm

(56) References cited:
- US-A- 4 010 755
- US-A- 4 013 081
- US-A- 4 444 195

## Description

The invention relates to an electrode apparatus, an intravascular or intracardiac pacemaker or defibrillation electrode in particular, with an electrode cable consisting of a sleeve of insulation enclosing a first elongated, flexible conductor, connected to a first electrode arranged on the electrode cable, and at least a second conductor, connected to a first conductive surface arranged on the electrode cable at a distance from the first electrode.

With the aid of a bipolar pacemaker electrode apparatus, the heart can be stimulated and cardiac activity sensed with the aid of the electrodes arranged on the electrode apparatus. In order to achieve good sensing of cardiac activity, the electrode for this purpose is generally placed at a distance from the stimulation electrode which, in most instances, is arranged at the distal end of the electrode cable. With an implanted intracardiac pacemaker electrode, the stimulation electrode usually presses against the heart wall in the inferior part of the ventricle. If the physician wishes to apply the sensing electrode in the ventricle, atrium or superior vena cava, he must select on each implantation occasion an electrode apparatus in which the distance between the stimulation electrode and the second electrode is such that the latter electrode can be applied at the desired site. Since the size and shape of the heart varies from one patient to another, the physician must have access to a large number of pacemaker electrode apparatuses with different distances between the said electrodes so she/he can select an electrode apparatus in which the distance between the electrodes permits optimal siting of the second electrode. Even with a pacemaker electrode apparatus for intravascular siting, the physician needs to select an electrode cable with an interelectrode distance suitable for the patient in order to achieve optimum sensing and stimulation of the heart.

In US-PS-5 172 694, a pacemaker electrode apparatus for intracardiac siting of the kind cited above is shown and described. By providing the electrode apparatus's cable with a plurality of relatively closely spaced consecutive indifferent electrodes, separated from the stimulation electrode, each with its own separate conductor, an attempt was made to reduce the number of electrode apparatuses which have to be stocked at a hospital. When the physician tests these electrodes, individually or in pairs, after the electrode apparatus has been implanted, she/he is able to identify the electrode or electrodes with the best position in the heart or vein and then disconnect the others. The disadvantage of this electrode apparatus is that only a limited number of indifferent electrodes can be arranged on the electrode cable, since the relatively thin cable must enclose a corresponding number of conductors. So no large stretch of cable can be covered with these electrodes, since the cable's dimensions, flexibility and connections would be adversely affected.

In US-PS-5 044 375, a defibrillation electrode apparatus for intravascular placement of the aforementioned kind is described. The defibrillation electrode apparatus contains two separately arranged defibrillation electrodes, in addition to a pacemaker stimulation electrode arranged at the distal end of the electrode cable, and a tightly spaced sensing electrode. With this electrode apparatus, the physician is unable to change the distance between the defibrillation electrodes.

The object of the invention is to achieve an electrode apparatus of the aforementioned kind in which the distance between the electrodes on the electrode cable can be changed with great simplicity and in which at least one electrode can be enlarged, reduced and, in certain embodiments, rotated around the electrode cable.

This problem is solved when the electrode apparatus is provided with at least one insulating sleeve-like body, which is slidable on the electrode cable, and at least partly covers the first conductive surface and with which a second electrode can be formed whose position in relation to the first electrode and/or whose size can be varied. With this structure for the electrode apparatus, the sleeve-like body or sleeve-like bodies, the second electrode can be slid along the first conductive surface in relation to the first electrode so the distance between the electrodes can be changed.

A pacemaker electrode apparatus according to the invention is used preferably in conjunction with DDD and VDD applications. "DDD" means that both the first and the second electrode are used for detection and stimulation, and "VDD" means that at least one electrode in the atrium senses atrial activity and synchronizes ventricular activity. In both these applications, one electrode is placed in the ventricle and the other in the atrium. Stimulation of the ventricle is inhibited when spontaneous activity is sensed so the electrode placed there has both a sensing and stimulating function. One or a plurality of electrodes placed in the atrium is/are often used for bipolar sensing of cardiac activity. So the ability to move this bipolar electrode, or these electrodes, along the electrode cable to an optimum position would therefore be highly advantageous.

The pacemaker electrode apparatus would also be useful for use in the ventricle, i.e. in which both electrodes are in the ventricle and the physician wishes to ascertain the propagation direction of a ventricular complex. The spontaneous complex starts from the wall of the septum, one-third of the way up from the cardiac apex. A different propagation direction is achieved when stimulation is with a stimulation electrode located at the apex. Knowledge of this can be used for distinguishing spontaneous heart contractions from stimulated contractions, with the aid of an electrode apparatus according to the invention whose electrode, not placed at the apex, can be moved to an optimum position in the ventricle.

In one advantageous embodiment of the invention, it is proposed that the second electrode be formed from the part of the first conductive surface which is not covered by the sleeve-like body. When the body is moved in relation to the said conductive surface, the electrode surface can be enlarged or reduced. In this way, the physician can also change the distance between electrodes by exposing the end of the conductive surface facing away from the first electrode or by exposing the second surface facing towards the first electrode.

As regards an advantageous embodiment of the invention, it is proposed that the sleeve-like body have at least one opening which is smaller than the size of the first conductive surface, whereby the opening can be rotated around and/or slid along the first conductive surface. With a body devised in this way, the conductive surface exposed through the opening and forming the electrode can be aimed in the desired direction by rotating the body around the cable's longitudinal axis. In this way, the electrode can achieve a favorable distribution of current in heart tissue both with a pacemaker and a defibrillation apparatus. The opening with a pacemaker electrode apparatus is preferably aimed at excitable tissue. If the sleeve-like body is equipped with fixation means, e.g. in the form of tines, near the electrode formed by the body, the ability to rotate the body so the fixation means are aimed at and affixed to heart wall would be highly advantageous. This rotation would also be advantageous when a defibrillation electrode apparatus is in contact with sensitive venous or heart tissue. With this placement, the body can be rotated so it serves as insulation against the said tissue.

According to another advantageous embodiment of the invention, the first conductive surface consists of a thin coating deposited on the insulation. This coating can be e.g. vapor-deposited so the electrode cable's flexibility is not impaired. According to a preferred feature of the invention, the first conductive surface can even be formed from part of the second conductor which is then stripped of insulation sleeve. The second conductive surface can consist of a long stretch of electrode cable. The main thing is for the sleeve-like body to cover this conductive surface, at least in part.

According to another preferred feature of the invention, the first conductive surface can even consist of at least two preferably ring-shaped sub-surfaces connected to a common conductor.

The electrode apparatus can also be advantageously equipped with two sleeve-like bodies which can slide in relation to each other and/or rotate in relation to each other. The conductive surface, which is delineated by the opposing ends of the sleeves, can be enlarged or reduced with the sleeve-like bodies. Each sleeve-like body can also be provided with an opening which can be aimed in different directions when the body is rotated around the cable by the physician.

According to a preferred embodiment of the invention, it is proposed that the interior walls of at least one of the sleevelike bodies be provided at least in part with a conductive coating, connected to an electrode arranged on the body, which forms the second electrode. This second electrode can be ringshaped, semi-ring shaped or have some other advantageous shape. The electrode can be arranged anywhere on the body, e.g. on one end of the body. In a defibrillation electrode apparatus, the electrode can also cover a large part or the entire body.

According to another preferred embodiment of the invention, it is proposed that the electrode apparatus be equipped with a third conductor, connected to a second conductive surface arranged on the electrode cable, inside the sleeve of insulation, whereby the first and the second conductive surfaces are subdivided into sub-surfaces separately spaced along the electrode cable. This electrode apparatus construction is for e.g. a multipolar pacemaker electrode apparatus.

In conjunction with an electrode apparatus equipped with subsurfaces, it is proposed according to a preferred feature of the invention that the sleeve-like body be provided with two openings, spaced at a distance corresponding to the distance between two adjacent subsurfaces, whereby the length of one opening is less than the distance between these sub-surfaces. The length of the opening ensures that no opening is able to expose two sub-surfaces simultaneously. When the body is slid along the cable in such a way that each opening exposes one conductive surface, the surfaces being electrically separated from each other, a movement of these electrodes and, accordingly, a change in the distance between them and the first electrode, are achieved when a multipolar pacemaker electrode apparatus is involved.

According to a preferred embodiment of the invention, it is proposed that the length of the sleeve-like body be such that the body's proximal end is accessible to the physician after the first electrode has been applied. In this embodiment, the physician is able to control the body even after an implantation and thereby work her/his way to the best position(s) for the second electrode or second electrodes respectively.

According to another advantageous embodiment of the invention, the proximal end of the body can even be equipped with control means with which the body can be slid along or rotated around the electrode cable. The body can therefore be relatively short but still controllable after the electrode apparatus has been implanted.

According to another preferred feature of the invention, the interior walls of the sleevelike bodies and/or the surface of the electrode cable on which the bodies slide can be provided with a coating of lubricant. This would facilitate the body's sliding and rotation.

The invention will now be described in greater detail, referring to FIGURES in the attached drawings in which

FIG. 1 is a section through a heart with a pacemaker electrode apparatus according to the invention applied therein.

FIG. 2 is a section through a heart with a partially applied defibrillation apparatus according to the invention.

FIGS. 3-12 show essential parts of the defibrillation and pacemaker apparatus which elucidate the invention in various embodiments.

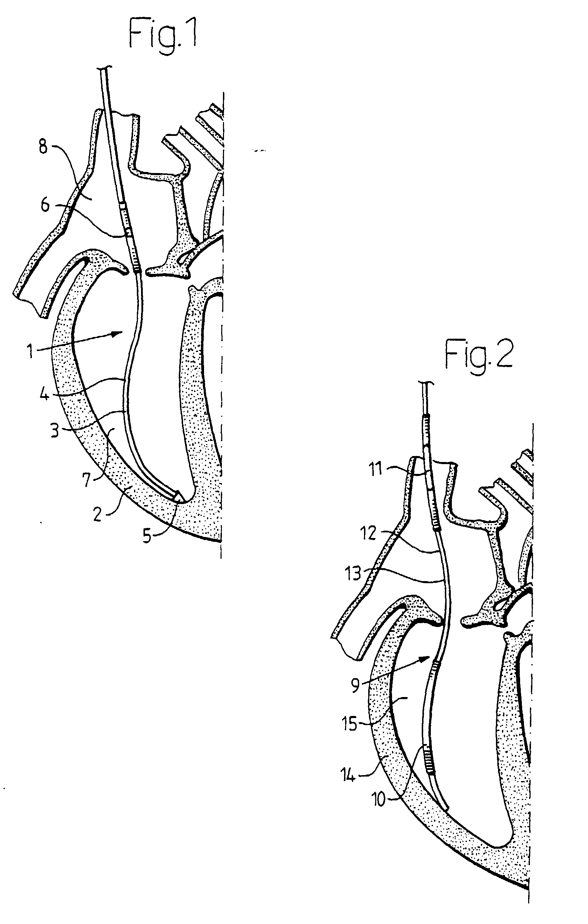

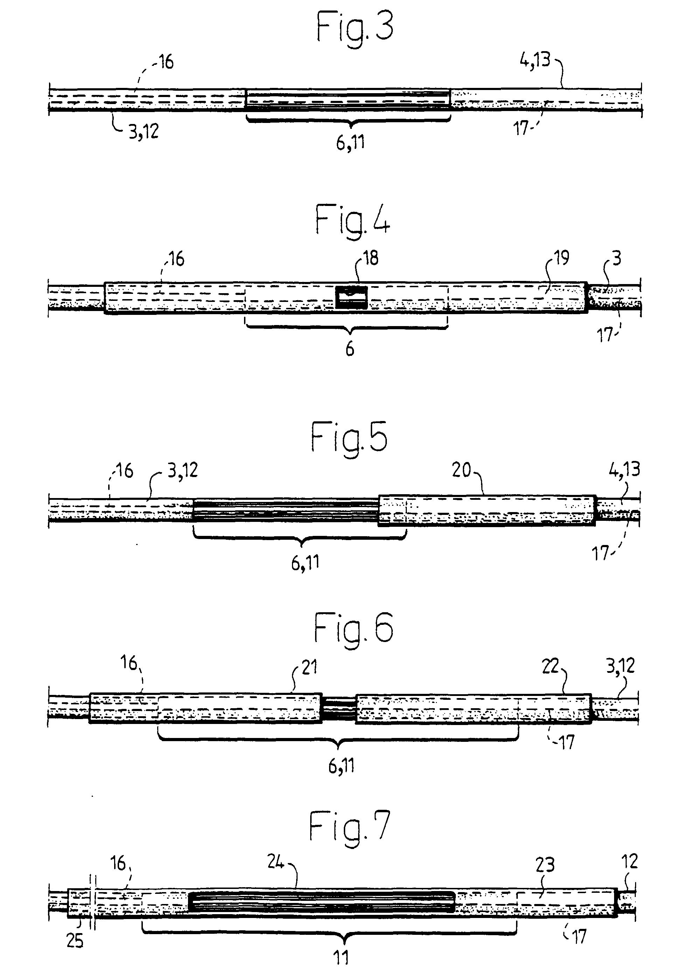

In FIG. 1 is shown a bipolar pacemaker electrode apparatus 1 which is applied in a patient's heart 2. The electrode apparatus 1 consists of an electrode cable 3 with a sleeve of insulation 4 enclosing a flexible conductor (not shown) connected to a stimulation electrode 5 on the distal end of the electrode cable 3. The sleeve of insulation 4 also encloses an additional conductor (also not shown in the FIGURE), connected to a conductive surface 6 on the electrode cable 3, forming a second e.g. indifferent electrode or sensing electrode. In this embodiment, the electrode 5 is anchored in the ventricle 7, and the second electrode 6 is floatingly arranged in the atrium 8. This means that the electrode 6 is not attached to the heart wall. The distance between the electrodes 5 and 6 can be varied with the pacemaker electrode apparatus 1 described here, as will be described in conjunction with several of the FIGURES 3-12.

In FIG. 2 is shown a defibrillation electrode apparatus 9, applied to a patient, consisting of an electrode cable 12 with a sleeve of insulation 13 enclosing two insulated conductors (not shown), one conductor of which connected to a first defibrillation electrode 10 on the electrode cable 12 and the second conductor connected to a conductive surface 11, serving as a second defibrillation electrode, on the electrode cable 12 at a distance from the defibrillation electrode 10. In this embodiment, the first defibrillation electrode 10 is located in the ventricle 15 of the heart 14, and the second defibrillation electrode 11 is arranged in the superior vena cava. The distance between the electrodes 10 and 11 for this defibrillation electrode apparatus 9 can be varied, as described in conjunction with several of the FIGURES 3-12.

In FIG. 3 is shown part of the electrode cable 3, 12 for the pacemaker or defibrillation electrode apparatus 1, 9 with the conductive surface 6, 11 covering a desired part of the sleeve of insulation 4, 13. The conductive surface 6, 11, which is connected to the aforementioned helical conductor 16 inside the sleeve of insulation 4, 13, is formed by a very thin coating of a conductive material deposited on the sleeve of insulation 4, 13 to enable the electrode cable 3, 12 to retain its flexibility. The conductive surface 6, 11 can also be formed from a part of the helical conductor 16, which is then stripped of insulation sleeve 4, 13. Here, the second conductor, connected to the first electrode 5 or 10, mentioned in conjunctions with FIGS. 1 and 2 bears the reference designations 17. In this FIG., the conductors 16, 17 are designated in stylized form with dashed lines.

In FIG. 4 is shown a sleeve-like body 19, made from an insulating material, and with a window-like opening 18, which can be slid along the electrode cable 3. The body 19 is arranged on the electrode cable 3 in such a way that it covers the conductive surface 6. Only the window 18 exposes part of this surface 6. When the body 19 is slid along the electrode cable 3, the window 18 is also displaced and, accordingly, the conductive surface 6 forming the second electrode in a bipolar pacemaker apparatus 1 in this embodiment. The distance between the electrode 5 and electrode 6 is also varied by the described displacement of the window 18. The length of the body 19 can preferably be such that the body covers the conductive surface 6 even when the window 18 has been moved to a position in which some peripheral part of the surface 6 forms the electrode.

In FIG. 5 the electrode cable 3, 12 for a pacemaker or defibrillation electrode apparatus 1, 9 is provided with a windowless, sleeve-like, flexible body 20. This embodiment shows that the body 20 can be moved in relation to the conductive surface 6, 11 in such a way that most of this surface 6, 11 is exposed. The surface 6, 11 can be enlarged or reduced when the body 20 is slid in one direction or the other. There is a simultaneous change in the distance between surface 6, 11 which serving as an electrode, and the first electrode 5, 10.

In FIG. 6, the electrode cable 3, 12 is provided with two windowless, flexible bodies 21, 22 which can be slid against each other along the electrode cable 3, 12 and along the conductive surface 6, 11 so a pacemaker or defibrillation electrode 6, 11 whose size and position are changeable can be achieved. The exposed conductive surface always serves as the electrode here.

The shift of the bodies 19, 22 described in conjunction with FIGS. 4-6 is always performed by the physician before the electrode apparatus 1, 19 is implanted.

FIG. 7 shows a sleeve-like body 23 arranged on the electrode cable 12. The body 23 is equipped with an elongated window 24 which accordingly exposes a conductive surface 11 which is very large compared to the window 18 shown and described in conjunction with FIG. 4, serving as a defibrillation electrode in this example. FIG. 7 also shows that the body 23 is long enough to make its proximal end 25 accessible to the physician, even after an electrode apparatus has been implanted. This accessibility can be very useful to the physician, since the window 24 can be rotated around its longitudinal axis, and the exposed conductive surface 11 can be aimed in the desired direction, achieving a favourable distribution of current in heart tissue. In this embodiment, the sleeve-like body 23 can also be slid along the electrode cable 12, thereby changing the distance between the electrodes 10 and 11.

The length of the body 23 described here can obviously be used in conjunction with several of the previously described embodiments, e.g. the body 19 described in FIG. 4, which could advantageously have the corresponding length.

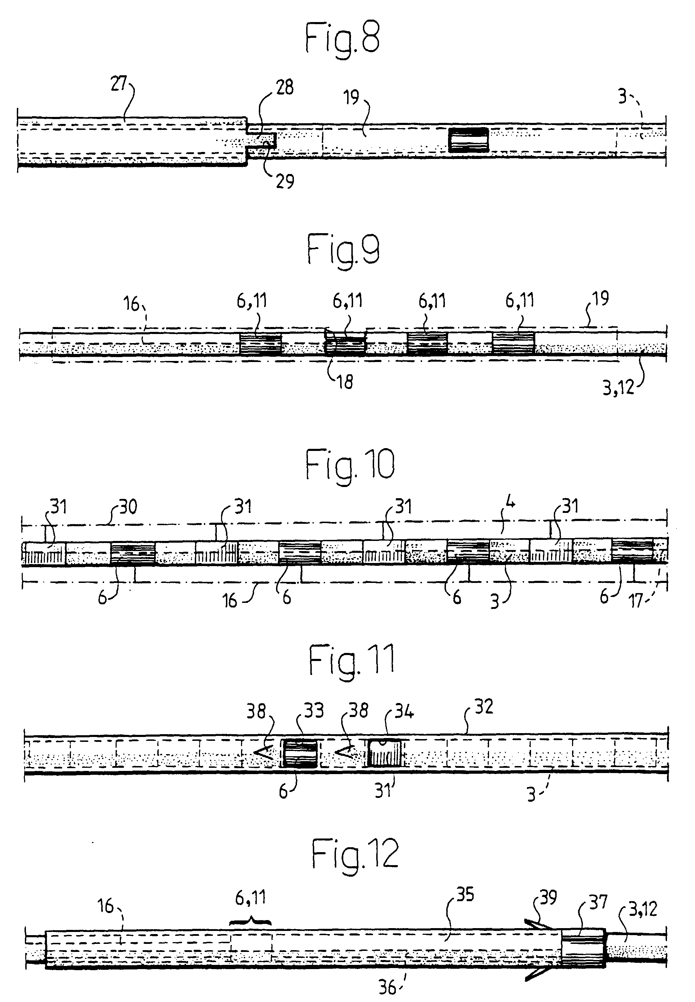

Another way to control a relatively short sleeve-like body, e.g. the body 19, with an implanted electrode apparatus is with the aid of a control device 27 is shown in FIG. 8. The control device 27 consists of a tubular, flexible part which is connectable to the body 19 and whose length is such that the physician is able to grasp the control device's 27 proximal end even after implantation. In this embodiment, the distal end of the control device 27 is equipped with a projecting part 28 which can be inserted into a corresponding recess 29 in the body 19. Using this control device 27, the physician would then be able to slide the body 19 along the electrode cable 3 and rotate the body 19 around its longitudinal axis. The means for connecting the distal end of the control device 27 to the body 19 could obviously be devised in other ways than the one shown in FIG. 8. If the physician does not wish to rotate the body 19 around its longitudinal axis but merely to slide it along the electrode cable 3, the control device 27 does not have to be equipped with gripping means 28, 29. The internal diameter of the tubular control device 27 is preferably somewhat larger than the external diameter of the electrode cable 3, thereby reducing friction between these parts.

In FIG. 9 is shown that the conductive surface 6, 11 can also consist of a plurality of consecutively spaced sub-surfaces connected to a common conductor 16. An electrode cable 3, 12 provided with such sub-surfaces can be advantageously used with a pacemaker electrode apparatus 1 in which e.g. the body 19 is slidable on the electrode cable 3 and in which the length of the window 18 roughly corresponds to the length of one sub-surface. In the FIG. the body 19 is designated with dashed lines. The FIG. also shows that the window 18 can be wider than has been shown for previous embodiments. Depending on the size of the sub-surfaces, an embodiment of the kind described here can also be used with a defibrillation electrode apparatus 9.

In FIG. 10 is shown a multipolar pacemaker electrode apparatus 1 in which the electrode cable 3, is equipped with a third conductor 30, arranged inside the sleeve of insulation and connected to a second conductive surface 31 arranged on the electrode cable, in addition to the previously described conductors 16, 17. The previously described first conductive surface 6 and the second conductive surface 31 are subdivided into sub-surfaces 6, 31 separately spaced along the electrode cable 3. For the sake of clarity, the conductors 16 and 30 are schematically rendered with dashed lines outside the electrode cable 3 with a connection to the respective sub-surfaces. This electrode cable 3 is preferably provided with a sleeve-like body 32 with two openings or windows 33, 34, consecutively spaced at a distance corresponding to the distance between two adjacent sub-surfaces 6, 31. One such sleeve-like body 35 is shown in FIG. 11. To prevent a window 33, 34 from simultaneously exposing parts of a first and second sub-surface 6 and 31, the length of a window 33, 34 is less than the distance between the sub-surfaces 6, 31. When the body 32 is slid along the electrode cable 3, the distance between the first electrode 5 and the sub-surfaces 6, 31 changes, thereby forming two additional electrodes. This embodiment shows that the sleeve-like body 32 is equipped with projecting fixation means in the form of tines 38 arranged by the windows 33, 34. With the aid of the fixation means, the body 32 can be attached to the heart wall so the sub-surfaces 6, 31 come into direct contact with excitable tissue, thereby resulting in a very low stimulation threshold.

In FIG. 12 is shown a sleeve-like body 35 which has been slid onto the electrode cable 3,12 for the pacemaker or defibrillation electrode apparatus 1, 9. The interior wall of the body 35 is provided with a conductive coating 36, e.g. in the form of a strip running along the entire length of the body 35, as shown in the FIG. The conductive coating 36 is connected to an electrode 37, arranged on top of the body 35, which forms the second electrode in one of the electrode apparatuses 1, 9. In this embodiment, the electrode 37 is arranged on one end of the body 35. As a result of the strip-shaped conductive coating 36, the electrode 37 is always in connection with the first conductive surface 6, 11 as long as the body 35 covers this surface 6, 11 on the electrode cable 3, 12. The electrode 37 can be arranged anywhere on top of the body and even have a larger area than the one shown in conjunction with this FIG. The electrode surface does not necessarily have to be ring-shaped. A relatively large conductive surface on the body 35 can serve as a defibrillation electrode. When the body is slid along the electrode cable 3, 12, the distance between this electrode and the first electrode 5, 10 can be enlarged or reduced. An electrode which is not ring-shaped can be aimed in the desired direction if the body is rotated on its longitudinal axis. The body 35 also has tines 39 which are arranged next to the electrode 37.

In order to facilitate sliding or rotation of the sleeve-like bodies 19-23, 32 and 35, their interior walls and/or the surface of the electrode cable 3, 12 on which the bodies slide are provided with a coating of lubricant not shown in the FIGS.

Within the scope of the invention the said first electrode 5 for the pacemaker electrode apparatus 1 and the electrode 10 for the defibrillation electrode apparatus can be constructed in the way described in conjunction with the second electrode/second electrodes respectively in the application. With the invention is achieved one or a plurality of electrodes which can be continuously slid along the electrode cable so these electrodes can be placed in the desired position in the patient. The size and aiming of the electrode can also be varied in some instances.

### Reference designations

- 1: Pacemaker electrode apparatus
- 2,14: Heart
- 3,12: Electrode cable
- 4,13: Sleeve of insulation
- 5: First electrode
- 6: First conductive surface, indifferent electrode
- 7,15: Ventricle
- 8: Atrium
- 9: Defibrillation electrode apparatus
- 10,11: Defibrillation electrode, first conductive surface, first electrode
- 16,17,30: Conductors
- 18,34,33,34: Opening, window
- 19,20: Sleeve-like body
- 21,22 23,32,35 25: Proximal end of the body
- 27: Control means, control device
- 28: Projecting part, gripping means
- 31: Second conductive surface
- 36: Conductive coating
- 37: Electrode
- 38: Tines

## Claims

1. An electrode apparatus (1, 9), an intravascular or intracardiac pacemaker or defibrillation electrode in particular, with an electrode cable (3) consisting of a sleeve of insulation (4) enclosing a first elongated, flexible conductor, connected to a first electrode (5) arranged on the electrode cable, and at least a second conductor, connected to a first conductive surface (6) arranged on the electrode cable at a distance from the first electrode, **characterized** in that the electrode apparatus (1, 9) is provided with at least one insulating sleeve-like body (19,23, 32, 35), which is slidable on the electrode cable (3, 12), and at least partly covers the first conductive surface (6, 11) and with which a second electrode (6, 11, 37) can be formed whose position in relation to the first electrode (5, 10) and/or whose size can be varied.

2. An electrode apparatus according to claim 1, **characterized** in that the second electrode (6, 11) is formed from the part of the first conductive surface (6, 11) which is not covered by the sleeve-like body (19-23, 32).

3. An electrode apparatus according to claim 1 or 2, **characterized** in that the sleeve-like body (19, 23 32) has at least one opening (18, 24, 33, 34) which is smaller than the size of the first conductive surface (6, 11), whereby the opening (18, 24, 33, 34) can be rotated around and/or slid along the first conductive surface (6, 11) .

4. An electrode apparatus according to any of claims 1 - 3, **characterized** in that the first conductive surface (6, 11) is formed from a thin coating deposited on the sleeve of insulation (4, 13).

5. An electrode apparatus according to any of claims 1 - 2, **characterized** in that the first conductive surface (6, 11) is formed from the part of the second conductor (16) which is stripped of insulation sleeve (4, 13).

6. An electrode apparatus according to any of claims 1 - 5, **characterized in** that the first conductive surface (6, 11) consists of at least two preferably ring-shaped sub-surfaces.

7. An electrode apparatus according to any of claims 1 - 6, **characterized in** that it is equipped with two sleeve-like bodies (21, 22) which can slide in relation to each other and/or rotate in relation to each other.

8. An electrode apparatus according to any of claims 4-7, **characterized in** that the interior walls of at least one of the sleeve-like bodies (35) is provided at least in part with a conductive coating (36), connected to an electrode (37) arranged on the body (35), which forms the second electrode.

9. An electrode apparatus according to claim 8, **characterized in** that the electrode (37) is arranged on one end of the body (35).

10. An electrode apparatus according to any of claims 1 - 9, **characterized in** that the electrode apparatus (1) is equipped with a third conductor (30), connected to a second conductive surface (31) arranged on the electrode cable (3), inside the sleeve of insulation (4), whereby the first and the second conductive surfaces (6, 31) are subdivided into sub-surfaces separately spaced along the electrode cable (3).

11. An electrode apparatus according to any of claims 1 - 7 and 10, **characterized in** that the sleeve-like body (32) is provided with two openings (33, 34), spaced at a distance corresponding to the distance between two adjacent sub-surfaces (6, 31), whereby the length of one opening (33, 34) is less than the distance between these sub-surfaces.

12. An electrode apparatus according to any of claims 1 - 11, **characterized** in that the length of the sleeve-like body (19, 23) is such that its proximal end (25) is accessible to the physician after the first electrode (5, 10) has been applied.

13. An electrode apparatus according to any of claims 1 - 12, **characterized** in that the proximal end of the body (19) is equipped with control means (27) with which the body (19) can be slid along or rotated around the electrode cable (3) .

14. An electrode apparatus according to any of claims 1 - 13, **characterized** in that the body (32, 37) is equipped with projecting fixation means (38, 39) which are preferably arranged near the opening or openings (33, 34) or by the electrode (37).

15. An electrode apparatus according to any of claims 1 - 14, **characterized** in that the interior walls of the sleeve-like bodies (19-23, 32, 35) and/or surface or the electrode cable (3, 12) on which the bodies slide are provided with a coating of lubricant.

## Patentansprüche

1. Eine Elektrodenvorrichtung (1, 9), insbesondere eine intravaskulare oder intrakardiale Herzschrittmacher- oder Defibrillationselektrode mit einem Elektrodenkabel, das aus einem Isolationsmantel (4) besteht, der einen ersten langgestreckten, flexiblen Leiter, der mit einer an dem Elektrodenkabel angeordneten ersten Elektrode (5) verbunden ist, und mindestens einen zweiten Leiter umschließt, der mit einer auf dem Elektrodenkabel in einem Abstand von der ersten Elektrode angeordneten, ersten leitenden Oberfläche (6) verbunden ist, **dadurch gekennzeichnet**, daß die Elektrodenvorrichtung (1, 9) mindestens mit einem isolierenden, hülsenförmigen Körper (19-23, 32, 35) versehen ist, der auf dem Elektrodenkabel (3, 12) verschiebbar ist und zumindest teilweise die erste leitende Oberfläche (6, 11) abdeckt und mit dem eine zweite Elektrode (6, 11, 37) gebildet werden kann, deren Lage in Bezug auf die erste Elektrode (5, 10) und/oder deren Größe variiert werden kann.

2. Eine Elektrodenvorrichtung nach Anspruch 1, **dadurch gekennzeichnet**, daß die zweite Elektrode (6, 11) aus dem Teil der ersten leitenden Oberfläche (6, 11), der nicht von dem hülsenförmigen Körper (19-23, 32) abgedeckt ist, gebildet wird.

3. Eine Elektrodenvorrichtung nach Anspruch 1 oder 2, **dadurch gekennzeichnet**, daß der hülsenförmige Körper (19, 23, 32) zumindest eine Öffnung (18, 24, 33, 34) aufweist, die kleiner als die Größe der ersten leitenden Oberfläche (6, 11) ist, wobei die Öffnung (18, 24, 33, 34) um die erste leitende Oberfläche (6, 11) gedreht und/oder entlang der ersten leitenden Oberfläche (6, 11) verschoben werden kann.

4. eine Elektrodenvorrichtung nach einem der Ansprüche 1 - 3, **dadurch gekennzeichnet**, daß die erste leitende Oberfläche (6, 11) durch eine dünne, auf den Isolationsmantel (4, 13) aufgetragene Beschichtung gebildet wird.

5. Eine Elektrodenvorrichtung nach einem der Ansprüche 1 - 2, **dadurch gekennzeichnet,** daß die erste leitende Oberfläche (6, 11) durch den Teil des zweiten Leiters (16) gebildet wird, von dem der Isolationsmantel (4, 13) entfernt wurde.

6. Eine Elektrodenvorrichtung nach einem der Ansprüche 1 - 5, **dadurch gekennzeichnet**, daß die erste leitende Oberfläche (6, 11) aus mindestens zwei vorzugsweise ringförmigen Unteroberflächen besteht.

7. Eine Elektrodenvorrichtung nach einem der Ansprüche 1 - 6, **dadurch gekennzeichnet**, daß sie mit zwei hülsenförmigen Körpern (21, 22), die in Beziehung zueinander verschoben und/oder gedreht werden können, ausgestattet ist.

8. Eine Elektrodenvorrichtung nach einem der Ansprüche 4 - 7, **dadurch gekennzeichnet**, daß die Innenwände zumindest eines der hülsenförmigen Körper (35) zumindest teilweise mit einer leitenden Beschichtung (36) versehen sind, die mit einer Elektrode (37), die auf dem Körper (35) angeordnet ist und die zweite Elektrode bildet, verbunden ist.

9. Eine Elektrodenvorrichtung nach Anspruch 8, **dadurch gekennzeichnet**, daß die Elektrode (37) an einem Ende des Körpers (35) angeordnet ist.

10. Eine Elektrodenvorrichtung nach einem der Ansprüche 1 - 9, **dadurch gekennzeichnet**, daß die Elektrodenvorrichtung (1) mit einem dritten Leiter (30) ausgestattet ist, der mit einer auf dem Elektrodenkabel (3) innerhalb des Isolationsmantels (4) angeordneten, zweiten leitenden Oberfläche (31) verbunden ist, wobei die erste und die zweite leitende Oberfläche (6, 31) in separat entlang des Elektrodenkabels (3) verteilte Unteroberflächen unterteilt sind.

11. Eine Elektrodenvorrichtung nach einem der Ansprüche 1 - 7 und 10, **dadurch gekennzeichnet**, daß der hülsenförmige Körper (32) mit zwei Öffnungen (33, 34) versehen ist, die mit einem zu dem Abstand zwischen zwei benachbarten Unteroberflächen (6, 31) korrespondierenden Zwischenraum angeordnet sind, wobei die Länge einer Öffnung (33, 34) kleiner ist als der Abstand zwischen diesen Unteroberflächen.

12. Eine Elektrodenvorrichtung nach einem der Ansprüche 1 - 11, **dadurch gekennzeichnet**, daß die Länge des hülsenförmigen Körpers (19, 23) so ist, daß sein proximales Ende (25) für den Arzt zugänglich ist, nachdem die erste Elektrode (5, 10) angebracht wurde.

13. Eine Elektrodenvorrichtung nach einem der Ansprüche 1 - 12, **dadurch gekennzeichnet,** daß das proximale Ende des Körpers (19) mit Steuermitteln (27) ausgestattet ist, mit denen der Körper (19) entlang dem Elektrodenkabel (3) verschoben oder um dieses herum gedreht werden kann.

14. Eine Elektrodenvorrichtung nach einem der Ansprüche 1 - 13, **dadurch gekennzeichnet,** daß der Körper (32, 37) mit vorspringenden Fixierungsmitteln (38, 39) ausgestattet ist, die vorzugsweise nahe der Öffnung oder der Öffnungen (33, 34) oder der Elektrode (37) angeordnet sind.

15. Einen Elektrodenvorrichtung nach einem der Ansprüche 1 - 14, **dadurch gekennzeichnet,** daß die Innenwände der hülsenförmigen Körper (19 - 23, 32, 35) und/oder die Oberfläche des Elektrodenkabels (3, 12), auf dem die Körper gleiten, mit einer Beschichtung aus einem Schmiermittel versehen sind.

## Revendications

1. Dispositif (1, 9) d'électrode, stimulateur cardiaque, intracardiaque ou intravasculaire ou électrode de défibrillation notamment, ayant un câble (3) d'électrode constitué d'un manchon (4) d'isolation enfermant un premier conducteur oblong souple, relié à une première électrode (5) disposée sur le câble d'électrode, et au moins un second conducteur, relié à une première surface (6) conductrice disposée sur le câble d'électrode à distance de la première électrode, caractérisé en ce que le dispositif (1,9) d'électrode est muni d'au moins un corps (19 à 23, 32, 35) en forme de manchon isolant, qui peut coulisser sur le câble (3, 12) d'électrodes et au moins partiellement recouvrir la première surface (6, 11) conductrice et avec lequel il peut être formée une seconde électrode (6, 11, 37) dont la position par rapport à la première électrode (5, 10) et/ou dont la dimension peut être modifiée.

2. Dispositif d'électrode suivant la revendication 1, caractérisé en ce que la seconde électrode (6, 11) est formée de la partie de la première surface (6, 11) conductrice qui n'est pas recouverte par le corps (19-23, 32) en forme de manchon.

3. Dispositif d'électrode suivant la revendication 1 ou 2, caractérisé en ce que le corps (19 à 23, 32) en forme de manchon a au moins une ouverture (18, 24, 33, 34) qui est plus petite que la dimension de la première surface (6, 11) conductrice, grâce à quoi l'ouverture (18, 24, 33, 34) peut être tournée par rapport à la première surface (6, 11) conductrice ou coulisser le long de la première surface (6, 11) conductrice.

4. Dispositif d'électrodes suivant l'une quelconque des revendications 1 à 3, caractérisé en ce que la première surface (6, 11) conductrice est formée d'un revêtement mince déposé sur le manchon d'isolation (4, 13).

5. Dispositif d'électrode suivant l'une quelconque des revendications 1 ou 2, caractérisé en ce que la première surface (6, 11) conductrice est formée de la partie du second conducteur (16) qui est enrubanné du manchon (4, 13) d'isolation.

6. Dispositif d'électrode suivant l'une quelconque des revendications 1 à 5, caractérisé en ce que la première surface (6, 11) conductrice est constituée d'au moins deux sous-surfaces préférablement en forme de bague.

7. Dispositif d'électrode suivant l'une quelconque des revendications 1 à 6, caractérisé en ce qu'il est muni d'au moins deux corps (21, 22) en forme de manchon, qui peuvent coulisser l'un par rapport à l'autre et/ou tourner l'un par rapport à l'autre.

8. Dispositif d'électrode suivant l'une quelconque des revendications 4 à 7, caractérisé en ce que les parois intérieures d'au moins l'un des corps (35) en forme de manchon sont munies au moins en partie d'un revêtement (36) conducteur, relié à une électrode (37) prévue sur le corps (35), qui forme la seconde électrode.

9. Dispositif d'électrode suivant la revendication 8, caractérisé en ce que l'électrode (37) est disposée à une extrémité du corps (35).

10. Dispositif d'électrode suivant l'une quelconque des revendications 1 à 9, caractérisé en ce que le dispositif (1) d'électrode est muni d'un troisième conducteur (30), relié à une seconde surface (31) conductrice disposée sur le câble (3) d'électrode, à l'intérieur du manchon d'isolation (4), grâce à quoi les première et seconde surfaces (6, 31) conductrices sont subdivisées en des sous-surfaces espacées en étant séparées le long du câble (3) d'électrode.

11. Dispositif d'électrode suivant l'une quelconque des revendications 1 à 7 et 10, caractérisé en ce que le corps (32) en forme de manchon est muni de deux ouvertures (33, 34), à distance l'une de l'autre correspondant à la distance entre deux sous-surfaces (6, 31) adjacentes, grâce à quoi la longueur d'une ouverture (33, 34) est moindre que la distance entre ces sous-surfaces.

12. Dispositif d'électrode suivant l'une quelconque des revendications 1 à 11, caractérisé en ce que la longueur du corps (19, 23) en forme de manchon est telle que son extrémité (25) proximale est accessible au médecin après que la première électrode (5, 10) a été appliquée.

13. Dispositif d'électrode suivant l'une quelconque des revendications 1 à 12, caractérisé en ce que l'extrémité proximale du corps (19) est munie de moyens (27) de commande, par lesquels on peut faire coulisser le corps (19) le long du câble (3) d'électrode ou le faire tourner autour du câble (3) d'électrode.

14. Dispositif d'électrode suivant l'une quelconque des revendications 1 à 13, caractérisé en ce que le corps (32, 37) est muni de moyens (38, 39) de fixation faisant saillie, qui sont disposés de préférence à proximité de l'ouverture ou des ouvertures (33, 34) ou à l'électrode (37).

15. Dispositif d'électrode suivant l'une quelconque des revendications 1 à 14, caractérisé en ce que les parois intérieures des corps (19-23, 32, 35) en forme de manchon et/ou de la surface ou du câble (3, 12) d'électrode sur lesquelles les corps coulissent sont munies d'un revêtement de lubrifiant.
